# EUROPEAN PATENT SPECIFICATION

(11) **EP 3 996 712 B1**
(45) Date of publication and mention of the grant of the patent: **18.03.2026**
(21) Application number: 20841310.4
(22) Date of filing: 12.07.2020
(51) Int. Cl.: A61K 31/4439, A61K 31/4545, A61K 31/47, A61P 25/28, A61K 31/519

(54) **P38ALPHA MAPK INHIBITORS FOR USE IN THE TREATMENT OF DEMENTIA WITH LEWY BODIES**
P38ALPHA-MAPK-INHIBITOREN ZUR VERWENDUNG BEI DER BEHANDLUNG VON DEMENZ MIT LEWY-KÖRPERCHEN
INHIBITEURS DE LA P38ALPHA MAPK UTILISÉS DANS LE TRAITEMENT DE LA DÉMENCE À CORPS DE LEWY

(30) Priority: 12.07.2019 US 201962873813 P
(43) Date of publication of application: 18.05.2022
(73) Proprietor: EIP Pharma, Inc., Boston, MA 02116 (US)
(72) Inventor: ALAM, John, Jahangir, Boston, MA 02116 (US)
(74) Representative: Casalonga
(86) International application number: PCT/US2020/041736
(87) International publication number: WO 2021/011432

(56) References cited:
- WO-A1-2016/007616
- WO-A1-2017/185073
- US-B2- 9 539 259
- LEE GARAM ET AL: "Clinical drug development for dementia with Lewy bodies: past and present", EXPERT OPINION ON INVESTIGATIONAL DRUGS, vol. 28, no. 11, 28 October 2019 (2019-10-28), UK, pages 951 - 965, XP093217669, ISSN: 1354-3784, DOI: 10.1080/13543784.2019.1681398
- "Cognitive Effects of Oral p38 Alpha Kinase Inhibitor Neflamapimod in Dementia With Lewy Bodies (AscenD-LB", CLINICALTRIALS.GOV, 28 June 2019 (2019-06-28), XP055786939, Retrieved from the Internet <URL:https://clinicaltrials.gov/ct2/show/NCT04001517> [retrieved on 20200925]
- TERRY MARK: "EIP Pharma Closes $20.5 to Fund Alzheimer's Studies", BIOSPACE, 16 May 2018 (2018-05-16), pages 1 - 4, XP055786945, Retrieved from the Internet <URL:https://www.biospace.com/article/eip-pharma-closes-20-5-to-fund-alzheimer-s-studies/#:~:text=EIP%20Pharma%2C%20based%20in%20Cambridge,the%20treatment%20of%20Alzheimer's%20disease.> [retrieved on 20200925]
- POWER JOHN H T , BARNES OLIVIA L., CHEGINI FARIBA: "Lewy Bodies and the Mechanisms of Neuronal Cell Death in Parkinson's Disease and Dementia with Lewy Bodies", BRAIN PATHOL, vol. 27, no. 1, 2017 - 18 January 2016 (2016-01-18), pages 3 - 12, XP055786950, DOI: 10.1111/bpa.12344
- ALAM ET AL.: "Neflamapimod: Clinical Phase 2b-Ready Oral Small Molecule Inhibitor of p38a to Reverse Synaptic Dysfunction in Early Alzheimer's Disease", J PREV ALZ DIS 2017, vol. 4, no. 4, 27 September 2017 (2017-09-27), pages 273 - 278, XP009518393, Retrieved from the Internet <URL:http://dx.doi.org/10.14283/jpad.2017.41> DOI: 10.14283/jpad.2017.41
- ADAMOWICZ DAVID H., ROY SUBHOJIT, SALMON DAVID P., GALASKO DOUGLAS R., HANSEN LAWRENCE A., MASLIAH ELIEZER, GAGE FRED H.: "Hippocampal a-Synuclein in Dementia with Lewy Bodies Contributes to Memory Impairment and Is Consistent with Spread of Pathology", THE JOURNAL OF NEUROSCIENCE, vol. 37, no. 7, 15 February 2017 (2017-02-15), pages 1675 - 1684, XP055786953, DOI: 10.1523/JNEUROSCI.3047-16.2016
- FERMAN TANIS J., BOEVE BRADLEY F.: "Dementia with Lewy bodies", NEUROL CLIN, vol. 25, no. 3, August 2007 (2007-08-01), pages 741 - 760, XP055786957, DOI: 10.1016/j.ncl.2007.03.001
- NAVA-MESA MAURICIO O., JIMENEZ-DÃAZ LYDIA, YAJEYA JAVIER, NAVARRO-LOPEZ JUAN D.: "GABAergic neurotransmission and new strategies of neuromodulation to compensate synaptic dysfunction in early stages of Alzheimer's disease", FRONTIERS IN CELLULAR NEUROSCIENCE, vol. 8, no. Article 167, 25 June 2014 (2014-06-25), XP055786968, DOI: 10.3389/fncel.2014.00167

## Description

### BACKGROUND

Dementia with Lewy bodies (DLB) is the second most common dementia after Alzheimer's disease and yet there are no approved therapies to address this progressive disorder.

WO 2017/185073 A1 discloses methods and compositions for improving episodic memory and treating dementia. WO 2016/007616 A1 discloses methods for treating a neurological disorder comprising administering to a patient in need thereof a dose of VX-745, or a pharmaceutically acceptable composition thereof. "'Cognitive Effects of Oral p38 Alpha Kinase Inhibitor Neflamapimod in Dementia With Lewy Bodies (AscenD-LB', CLINICALTRIALS.GOV, 28 June 2019 (2019-06-28), XP055786939", discloses a double-blind, placebo-controlled 16-week study of the cognitive effects of the oral P38 alpha kinase inhibitor Neflamapimod in Dementia with Lewy Bodies (DLB). "ALAM et al.: "Neflamapimod: Clinical Phase 2b-Ready Oral Small Molecule Inhibitor of p38a to Reverse Synaptic Dysfunction in Early Alzheimer's Disease", J Prev Alz Dis 2017, vol. 4, no. 4 27 September 2017 (2017-09-27), pages 273-278, XP009518393, DOI: 10.14283/jpad.2017.41" discloses Neflamapimod/VX-745 and its use in the treatment of Alzheimer's disease.

### SUMMARY

The present disclosure encompasses the discovery that a selective p38 α mitogen activated protein kinase (MAPK) inhibitor can be used to prevent, reverse, or inhibit loss of cholinergic neuron input to the hippocampus. In particular, it has been found that treatment with the selective p38 α mitogen activated protein kinase (MAPK) inhibitor, neflamapimod, can improve the survival of neurons cholinergic neurons in the medial septum, a brain region underlying the pathological sequelae of Dementia with Lewy Bodies (DLB).

The invention is set out in the appended set of claims.

In the following, any reference to a method of treating a disease or disorder by administering a compound or composition is to be understood to refer to the compound or composition for use in the treatment of said disease or disorder.

Generally, herein disclosed is a method of treating a subject having Dementia with Lewy Bodies (DLB), the method comprising administering to the subject a selective p38α mitogen activated protein kinase (MAPK) inhibitor.

Also disclosed herein is a method of reversing synaptic dysfunction associated with alpha-synuclein in a subject, the method comprising administering to the subject a selective p38α mitogen activated protein kinase (MAPK) inhibitor.

Also disclosed herein is a method of treating alpha-synuclein associated degenerative disease in a subject, the method comprising administering to the subject a selective p38α mitogen activated protein kinase (MAPK) inhibitor.

Also disclosed herein is a method for inhibiting neuronal loss in the central nervous system of a subject, the method comprising administering to the subject a selective p38α mitogen activated protein kinase (MAPK) inhibitor.

Also disclosed herein is a method for reversing endosomal dysfunction in a subject having DLB, the method comprising administering to the subject a selective p38α mitogen activated protein kinase (MAPK) inhibitor.

In some embodiments, the selective p38α mitogen activated protein kinase (MAPK) inhibitor is neflamapimod.

In some embodiments, the synaptic dysfunction comprises dysfunction in the medial septum.

In some embodiments, the neuronal cell loss is in the hippocampus. In some embodiments, the neuronal cell loss is in CA2-3 regions of the hippocampus. In some embodiments, the neuronal cell loss is in the medial septum. In some embodiments, the neuronal cell loss is in the vertical limb of the nucleus of the diagonal band. In some embodiments, the neuronal cells are cholinergic neurons.

In some embodiments, a subject to be treated has alpha synuclein deposits in the hippocampus.

### BRIEF DESCRIPTION OF THE DRAWINGS

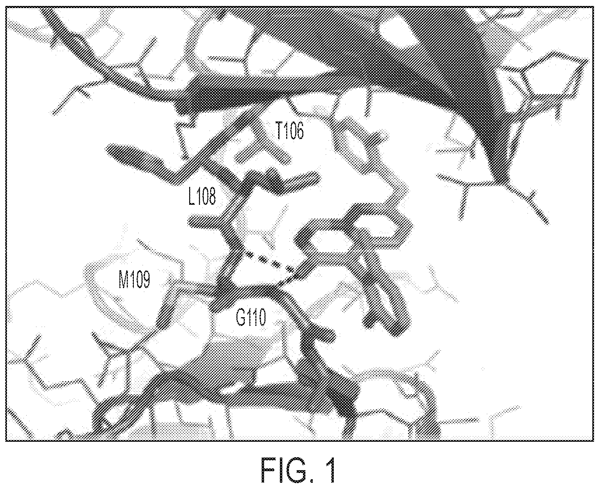

**Figure 1** shows the crystal structure of neflamapimod (VX-745), a specific inhibitor of p38α kinase activity.

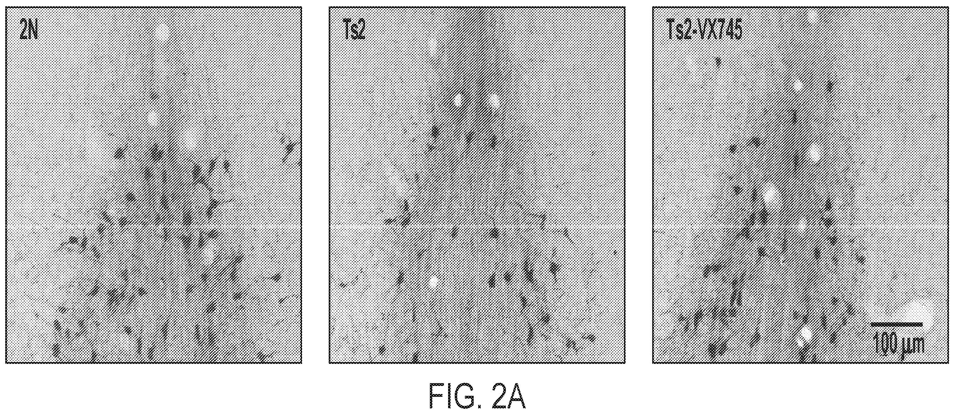

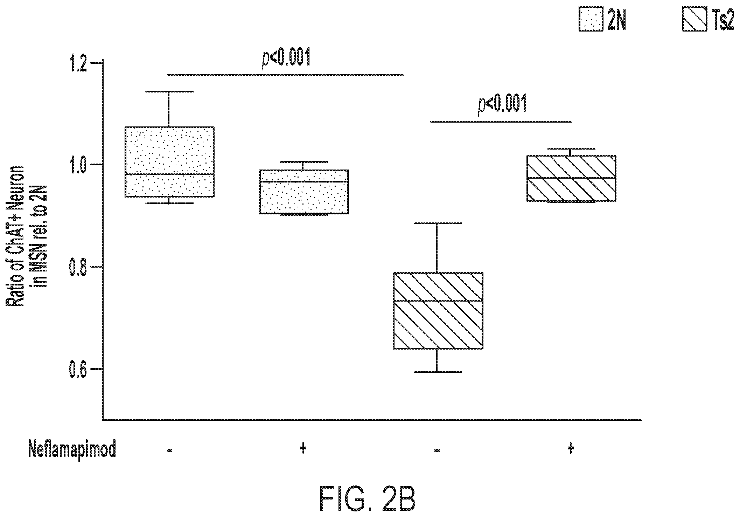

**Figures 2A-2B** show oral administration of neflamapimod toTs2 mice normalizes ChAT-positive neuron number in the medial septal nucleus. Shown are representative images of ChAT+ neurons from medial septal nucleus, and their stereological quantification, n=7-9 mice of each condition "+" indicating means; One-way ANOVA, *** P<0.005

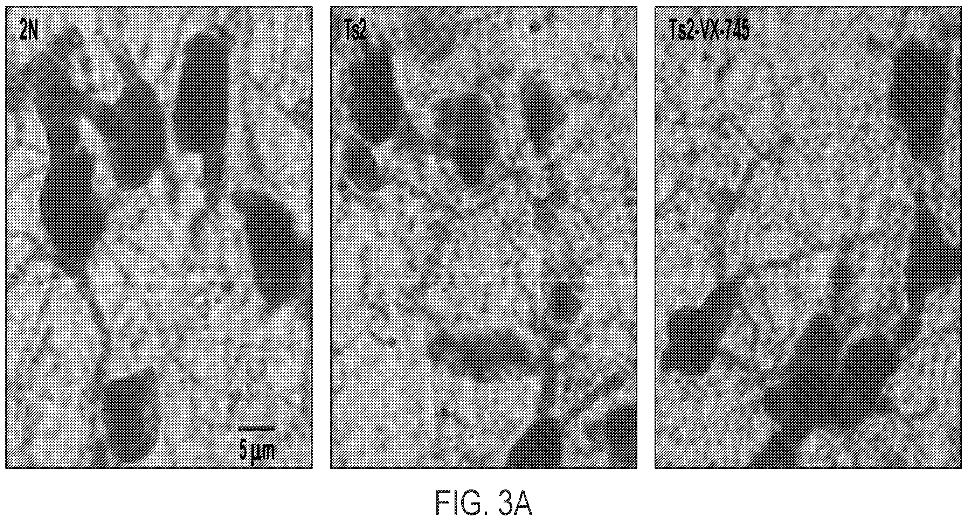

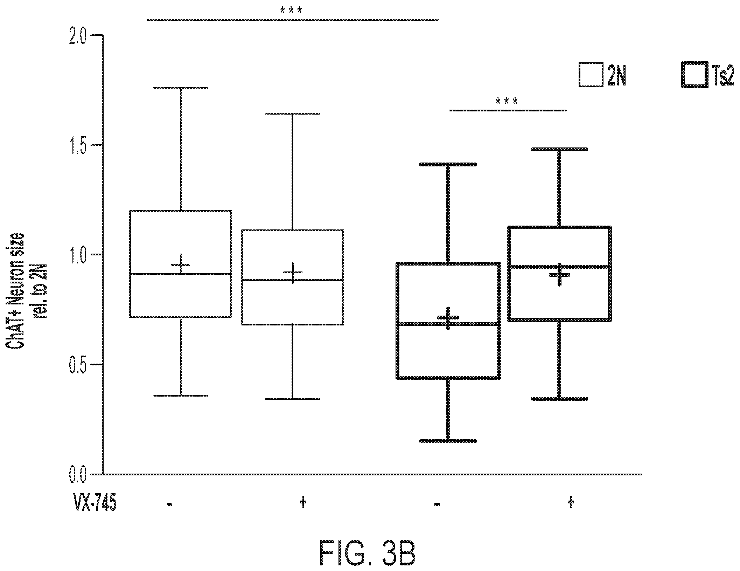

**Figures 3A-3B** show oral administration of neflamapimod normalizes morphology (size) in the medial sepal nucleus (MSN). Shown are representative images of ChAT+ neurons from medial septal nucleus, and their size quantification, n>99 ChAT+ neuron of each condition, "+" indicating means; One-way ANOVA, *** P<0.005

### DEFINITIONS

***Carrier:*** The term "carrier" refers to any chemical entity that can be incorporated into a composition containing an active agent *(e.g.,* a p38 MAPKα inhibitor such as neflamapimod) without significantly interfering with the stability and/or activity of the agent (*e.g.*, with a biological activity of the agent). In certain embodiments, the term "carrier" refers to a pharmaceutically acceptable carrier.

***Formulation.*** The term "formulation" as used herein refers to a composition that includes at least one active agent *(e.g.,* p38 MAPKα inhibitor such as neflamapimod) together with one or more carriers, excipients or other pharmaceutical additives for administration to a patient. In general, particular carriers, excipients and/or other pharmaceutical additives are selected in accordance with knowledge in the art to achieve a desired stability, release, distribution and/or activity of active agent(s) and which are appropriate for the particular route of administration.

***Pharmaceutically acceptable carrier, adjuvant, or vehicle.*** The term "pharmaceutically acceptable carrier, adjuvant, or vehicle" refers to a non-toxic carrier, adjuvant, or vehicle that does not destroy the pharmacological activity of the compound with which it is formulated. Pharmaceutically acceptable carriers, adjuvants or vehicles that may be used in the compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

***Therapeutically effective amount*** and ***effective amount.*** As used herein, and unless otherwise specified, the terms "therapeutically effective amount" and "effective amount" of an agent refer to an amount sufficient to provide a therapeutic benefit in the treatment, prevention and/or management of a disease, disorder, or condition, *e.g.,* to delay onset of or minimize (e.g., reduce the incidence and/or magnitude of) one or more symptoms associated with the disease, disorder or condition to be treated. In some embodiments, a composition may be said to contain a "therapeutically effective amount" of an agent if it contains an amount that is effective when administered as a single dose within the context of a therapeutic regimen. In some embodiments, a composition may be said to contain a "therapeutically effective amount" of an agent if it contains an amount that is effective when administered as more than one dose (e.g., two doses, three doses, or four or more doses) within the context of a therapeutic regimen. In some embodiments, a therapeutically effective amount is an amount that, when administered as part of a dosing regimen, is statistically likely to delay onset of or minimize (reduce the incidence and/or magnitude of) one or more symptoms or side effects of a disease, disorder or condition.

***Treat or Treating.*** The terms "treat" or "treating," as used herein, refer to partially or completely alleviating, inhibiting, delaying onset of, reducing the incidence of, yielding prophylaxis of, ameliorating and/or relieving or reversing a disorder, disease, or condition, or one or more symptoms or manifestations of the disorder, disease or condition.

***Unit Dose.*** The expression "unit dose" as used herein refers to a physically discrete unit of a formulation appropriate for a subject to be treated *(e.g.,* for a single dose); each unit containing a predetermined quantity of an active agent selected to produce a desired therapeutic effect when administered according to a therapeutic regimen (it being understood that multiple doses may be required to achieve a desired or optimum effect), optionally together with a pharmaceutically acceptable carrier, which may be provided in a predetermined amount. The unit dose may be, for example, a volume of liquid *(e.g.,* an acceptable carrier) containing a predetermined quantity of one or more therapeutic agents, a predetermined amount of one or more therapeutic agents in solid form *(e.g.,* a tablet or capsule), a sustained release formulation or drug delivery device containing a predetermined amount of one or more therapeutic agents, *etc.* It will be appreciated that a unit dose may contain a variety of components in addition to the therapeutic agent(s). For example, acceptable carriers (*e.g.*, pharmaceutically acceptable carriers), diluents, stabilizers, buffers, preservatives, *etc.,* may be included as described *infra.* It will be understood, however, that the total daily usage of a formulation of the present invention will be decided by the attending physician within the scope of sound medical judgment. The specific effective dose level for any particular subject may depend upon a variety of factors including the disorder being treated and the severity of the disorder; activity of specific active compound employed; specific composition employed; age, body weight, general health, sex and diet of the subject; time of administration, and rate of excretion of the specific active compound employed; duration of the treatment; drugs and/or additional therapies used in combination or coincidental with specific compound(s) employed, and like factors well known in the medical arts. In some embodiments, a unit dose of a p38 MAPKα inhibitor, such as neflamapimod is about 1 mg, 3 mg, 5 mg, 10 mg, 15 mg, 20 mg, 25 mg, 30 mg, 35 mg, 40 mg, 45 mg, 50 mg, 100 mg, 125 mg, or 250 mg.

### DETAILED DESCRIPTION OF CERTAIN EMBODIMENTS

The invention is set out in the appended set of claims.

Generally, the present invention provides, among other things, compositions and methods for treating Dementia with Lewy Bodies (DLB) and associated pathology, by administering a composition comprising a selective p38 MAPKα inhibitor. In some embodiments, the selective p38 MAPKα inhibitor is neflamapimod.

Also disclosed herein are compositions and methods for treating subjects susceptible or at risk of development or progression of DLB.

Various aspects of the invention are described in detail in the following sections. The use of sections is not meant to limit the invention. Each section can apply to any aspect of the invention. In this application, the use of "or" means "and/or" unless stated otherwise.

### Dementia With Lewy Bodies

There is currently no therapy available for DLB that reverses and/or slows disease progression. A therapeutic intervention such as neflamapimod, that targets synaptic dysfunction, has the potential to both reverse existing synaptic deficits and slow further decline.

The central feature of DLB is a progressive dementia, i.e. decline in cognition associated with functional deficits, that is characterized by deficits in attention and executive function but can include memory deficits. Associated symptoms include fluctuation in attentiveness, slowness of movement, rigidity, REM sleep disruption, visual hallucinations, anosmia, fluctuation in attentiveness, depression, apathy, and autonomic nervous system dysregulation. DLB is associate with deposits of alpha-synuclein in cells, known as Lewy bodies or Lewy neurites.

Hippocampal pathology is present in DLB, as well as AD, and memory impairment may be a prominent symptom of both disorders. However, the symptoms and pathology of DLB differs from AD in important ways. For example, in DLB, Lewy-related neurites can be found in CA2-CA3 regions of the hippocampus, a region which has been found at autopsy to be relatively preserved in AD (Fujishiro et al., Acta Neuropathol, 111:109-1114 (2006).

The medial septum (also known as Ch1) and the vertical limb of the diagonal band (also known as Ch2) provide cholinergic innervation to the hippocampus. Loss of neurons in the medial septum nucleus and vertrical limb of the diagonal band is a specific feature of DLB that differentiates it from AD (Fujishiro et al., Acta Neuropathol, 111:109-1114 (2006). It has been discovered herein that loss of cholinergic neurons in the medial septum can be inhibited by administration of a selective p38 α MAPK inhibitor, neflamapimod.

### Neflamapimod

Many extracellular stimuli, including pro-inflammatory cytokines and other inflammatory mediators, elicit specific cellular responses through the activation of mitogen-activated protein kinase (MAPK) signaling pathways. MAPKs are proline-targeted serine-threonine kinases that transduce environmental stimuli to the nucleus. Once activated, MAPKs activate other kinases or nuclear proteins through phosphorylation, including potential transcription factors and substrates. The four isoforms (α, β, δ, and γ) of p38 MAP kinase comprise one specific family of MAPKs that mediate responses to cellular stresses and inflammatory signals. Neflamapimod is a selective small-molecule inhibitor of the alpha isoform of p38 MAPK.

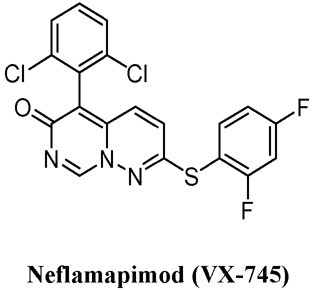

### Pharmaceutical Compositions

In some embodiments, a provided method comprises administering to a patient a pharmaceutical composition comprising neflamapimod together with one or more therapeutic agents and a pharmaceutically acceptable carrier, adjuvant, or vehicle. In some embodiments, the present invention provides a pharmaceutical composition comprising a dose of neflamapimod together with one or more therapeutic agents and a pharmaceutically acceptable carrier, adjuvant, or vehicle, wherein the dose of neflamapimod results in an average blood concentration of from about 1 ng/mL to about 15 ng/mL, from about 1 ng/mL to about 10 ng/mL, from about 5 ng/mL to about 15 ng/mL, or from about 5 ng/mL to about 10 ng/mL. In some embodiments, the dose of neflamapimod results in an average blood concentration of 8 ng/ml. Table 2 of WO 2017 /185073 illustrates neflamapimod plasma concentration values by post-dose collection time interval.

It should also be understood that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of a compound of the present invention in the composition will also depend upon the particular compound in the composition.

### Dosing

In some embodiments, a compositions are administered in a therapeutically effective amount and/or according to a dosing regimen that is correlated with a particular desired outcome (*e.g.*, with treating or reducing risk for disease).

In some embodiments, provided compositions are administered in a therapeutically effective amount and/or according to a dosing regimen that is correlated with a particular desired outcome *(e.g.,* reduction in , *etc*.).

Alternatively or additionally, in some embodiments, an appropriate dose or amount is determined through use of one or more *in vitro* or *in vivo* assays to help identify desirable or optimal dosage ranges or amounts to be administered.

In various embodiments, provided compositions are administered at a therapeutically effective amountGenerally, a therapeutically effective amount is sufficient to achieve a meaningful benefit to the subject (*e.g.*, treating, modulating, curing, preventing and/or ameliorating the underlying disease or condition). In some embodiments, a method of treating a subject having DLB comprise administering a therapeutically effective amount of a selective p38α inhibitor. In some embodiments, a method of treating a subject having DLB comprise administering a therapeutically effective amount of neflamapimod.

In some embodiments, a composition is provided as a pharmaceutical formulation. In some embodiments, a pharmaceutical formulation is or comprises a unit dose amount for administration in accordance with a dosing regimen correlated with achievement of disease reduction in symptoms of DLB, arrest or decrease in rate of decline of function due to DLB.

In some embodiments, a formulation comprising provided compositions as described herein is administered as a single dose. In some embodiments, a formulation comprising provided compositions as described herein is administered as two doses. In some embodiments, a formulation comprising provided compositions as described herein is administered at regular intervals. Administration at an "interval," as used herein, indicates that the therapeutically effective amount is administered periodically (as distinguished from a one-time dose). The interval can be determined by standard clinical techniques. In some embodiments, a formulation comprising provided compositions as described herein is administered twice weekly, thrice weekly, every other day, daily, twice daily, or every eight hours.

In some embodiments, a formulation comprising provided compositions as described herein is administered twice daily. In some embodiments, the twice daily administering occurs from about 9 to 15 hours apart. In some embodiments the twice daily administering occurs about 12 hours apart. In some embodiments, a formulation comprising from about 40 mg to about 250 mg of neflamapimod is administered twice daily. In some embodiments, the administering occurs when the patient is in a fed state. In some embodiments, the administering occurs within 30 to 60 minutes after the subject has consumed food. In some embodiments, the administering occurs when the patient is in a fasted state. The administration interval for a single individual need not be a fixed interval, but can be varied over time, depending on the needs of the individual.

In some embodiments, a formulation comprising provided compositions as described herein is administered at regular intervals. In some embodiments, a formulation comprising provided compositions as described herein is administered at regular intervals for a defined period. In some embodiments, a formulation comprising provided compositions as described herein is administered at regular intervals for 2 years, 1 year, 11 months, 10 months, 9 months, 8 months, 7 months, 6 months, 5 months, 4 months, 3 months, 2 months, a month, 3 weeks, 2, weeks, a week, 6 days, 5 days, 4 days, 3 days, 2 days or a day. In some embodiments, a formulation comprising provided compositions as described herein is administered at regular intervals for 16 weeks.

### EXEMPLIFICATION

The following examples are provided for illustrative purposes and are not intended to limit the scope of the invention.

### Example 1

Wild-type (WT) or Ts2 mice treated for 28 days, twice-daily, with vehicle (1%PluronicF108) or 3mg/kg neflamapimod in vehicle (n=8-10 per group; 1: 1 female/male). Treatment initiated at 4.7-6.4 months of age, when cholinergic neuronal loss is developing in Ts2 mice. Cortical Rab5+ endosomal number and size, and medial septal nucleus (MSN) cholineacetyltransferase (ChAT)+ neurons quantitated.

It was shown that neflamapimod treatment normalized ameliorated the cholinergic neuron degeneration in the medial septal nucleus of Ts2 mice.

### Example 2

### Neflamapimod treatment of human subjects having Dementia with Lewy Bodies (DLB)

A Phase 2, multi-center, randomized, double-blind, placebo-controlled study is run of neflamapimod versus matching placebo (randomized 1:1) administered with food for 16 weeks in subjects with DLB. The primary objective is to evaluate the effect of neflamapimod on cognitive function as assessed in a study-specific COGSTATE Neuropsychological Test Battery (NTB). Secondary endpoints include the Clinical Dementia Rating Scale-Sum of Boxes (CDR-SB), Mini-Mental State Examination (MMSE), Neuropsychiatric Inventory (NPI-10), Timed Up and Go Test, and electroencephalogram (EEG) as a potential biomarker for DLB.

Neflamapimod 40 mg capsules are administered orally, BID or TID with food for 16 weeks; subjects will follow the BID regimen if weighing <80 kg or the TID regimen if weighing ≥80 kg. A placebo comparator is 40 mg matching placebo capsules administered orally, BID or TID with food for 16 weeks; subjects will follow the BID regimen if weighing <80 kg or the TID regimen if weighing ≥80 kg.

A primary outcome measure is a composite score of a study-specific COGSTATE Neurological Test Battery (NTB) including the COGSTATE Letter Fluency Test and Category Fluency Test at 16 weeks. A change from Baseline to Week 16 in the composite score of a study-specific Cog-state Neurological Test Battery (NTB), including assessments of attention, executive function, and visuospatial function in neflamapimod treated-subjects as compared to the placebo-treated subjects, are analyzed using the Mixed Model Repeated Measures (MMRM) analysis method. The following six tests are included in the composite: (1) COGSTATE Detection test (DET), (2) COGSTATE Identification test (IDN), (3) COGSTATE One Card Learning test (OCL), (4) COGSTATE One Back test (ONB), (5) Letter Fluency Test, (6) Category Fluency Test (CFT). Each score on the individual tests will be converted to a z-score, and then a total z-score will be calculated, in which each test is weighted equally. The change in total z-score in neflamapimod vs. placebo-recipients is analyzed. As the analysis is based on z-scores, there is no minimum or maximum value.

A secondary outcome measure is a change in Clinical Dementia Rating Scale-Sum of Boxes (CDR-SB) score based on semi-quantitative scoring of each domain (box) evaluating cognitive impairment in milder and more progressive forms of dementia, in neflamapimod-treated subjects compared to placebo-recipients. The domain (box) scores is calculated for a Sum of Boxes score. Secondary efficacy endpoints utilize the same analysis method and model as the primary endpoint.

Another secondary outcome measure is a change in Mini-Mental State Examination (MMSE) with respect to orientation, memory, concentration, language, and praxis (scores ranging from 0 to 30 with lower scores indicating greater cognitive impairment), in neflamapimod-treated subjects compared to placebo-recipients. Secondary efficacy endpoints utilize the same analysis method and model as the primary endpoint.

Another secondary outcome measure is a change in International Shopping List Test (ISLT) immediate and delayed recall and recognition, which is used to assess episodic memory in neflamapimod-treated subjects compared to placebo-recipients. Secondary efficacy endpoints utilize the same analysis method as the primary endpoint.

Another secondary outcome measure is a change in Timed Up and Go Test (TUG) to assess mobility (score of >15 seconds indicates subject has increased risk of falls) in neflamapimod-treated subjects compared to placebo-recipients. Secondary efficacy endpoints utilize the same analysis method and model as the primary endpoint.

Another secondary outcome measure is a change in quantitative electroencephalogram (qEEG) parameters (all waveforms, with particular focus on relative alpha and theta power) with the subject awake in accordance with the 10-20 International System of Electrode placement is evaluated as a potential biomarker for DLB. Slowing of the dominant frequency band by qEEG over posterior aspects of the brain is recognized to be prominent in DLB, and various identified patterns may differentiate DLB from AD.

### Subjects

Inclusion criteria include the following:
Men and women aged ≥55 years.
Subject or subject's legally authorized representative is willing and able to provide written informed consent.
Probable DLB and identified cognitive deficits, according to current consensus criteria (McKeith et al, 2017), specifically one core clinical feature and a positive DaTscan. If a negative DaTscan, but the subject has historical PSG-verified RBD, the subject would also qualify.
MMSE score of 15-28, inclusive, during Screening.
Currently receiving cholinesterase inhibitor therapy, having received such therapy for greater than 3 months and on a stable dose for at least 6 weeks at the time of randomization. Except for reducing the dose for tolerability reasons, the dose of cholinesterase inhibitor may not be modified during the study.
Normal or corrected eye sight and auditory abilities, sufficient to perform all aspects of the cognitive and functional assessments.
No history of learning difficulties that may interfere with their ability to complete the cognitive tests.
Must have reliable informant or caregiver.

Exclusion criteria include the following:
Diagnosis of any other ongoing central nervous system (CNS) condition other than DLB, including, but not limited to, post-stroke dementia, vascular dementia, Alzheimer's disease (AD), or Parkinson's disease (PD).
Suicidality, defined as active suicidal thoughts within 6 months before Screening or at Baseline, defined as answering yes to items 4 or 5 on the C-SSRS, or history of suicide attempt in previous 2 years, or, in the Investigator's opinion, at serious risk of suicide.
Ongoing major and active psychiatric disorder and/or other concurrent medical condition that, in the opinion of the Investigator, might compromise safety and/or compliance with study requirements.
Diagnosis of alcohol or drug abuse within the previous 2 years.
Poorly controlled clinically significant medical illness, such as hypertension (blood pressure >180 mmHg systolic or 100 mmHg diastolic); myocardial infarction within 6 months; uncompensated congestive heart failure or other significant cardiovascular, pulmonary, renal, liver, infectious disease, immune disorder, or metabolic/endocrine disorders or other disease that would interfere with assessment of drug safety.
Aspartate aminotransferase (AST) or alanine aminotransferase (ALT) >2 × the upper limit of normal (ULN), total bilirubin >1.5 × ULN, and/or International Normalized Ratio (INR) >1.5.
Known human immunodeficiency virus, hepatitis B, or active hepatitis C virus infection.
Participated in a study of an investigational drug less than 3 months or 5 half-lives of an investigational drug, whichever is longer, before enrollment in this study.
History of previous neurosurgery to the brain.
If male with female partner(s) of child-bearing potential, unwilling or unable to adhere to contraception requirements specified in the protocol.
If female who has not has not reached menopause >1 year previously or has not had a hysterectomy or bilateral oophorectomy/salpingo-oophorectomy, has a positive pregnancy test result during Screening and/or is unwilling or unable to adhere to the contraception requirements specified in the protocol.

### Results

A total of 91 patients with mild or moderate dementia with Lewy bodies were enrolled and randomized between October 2019 and March 2020, 45 to receive 40 mg neflamapimod capsules and 46 to receive matching placebo capsules on a blinded basis. Patients were assigned to a twice daily (BID) or thrice daily (TID) dosing regimen, based on weight (BID if < 80 kg and TID if ≥ 80 kg).

The Covid-19 crisis had a significant impact on the conduct of the study, as between March and June 2020 many of the sites could not see the patients onsite at their respective clinical center; and instead monitored patients remotely via telephone or video chat, an approach which precluded obtaining the neuropsychological test battery (NTB; consisting of six cognitive tests) in approximately one-half of the visits during this timeframe. A final analysis using the Mixed Model for Repeated Measures (MMRM), with and without imputation of missing datapoints, may be employed to account for the effects of these remote visits.

A preliminary assessment revealed a robust dataset for evaluating efficacy at the Week 4 timepoint, with a significant drop-off in data availability beyond that timepoint. At the Week 4 timepoint a statistically significant positive neflamapimod treatment effect compared to placebo was seen for the COGSTATE Detection test (DET) and the Letter fluency test (LFT), measures of attention and executive function, respectively. For the DET, where a reduction represents improvement, the mean change from baseline to Week 4 was +0.024 in the placebo group versus -0.024 in neflamapimod group (p=0.031 for the difference, Wilcoxson rank sum test) (Table 1). For the LFT, where an increase represents improvement, the mean change from baseline to Week 4 was -1.7 in the placebo group versus +3.0 in neflamapimod group (p=0.027 for the difference) (Table 1). For both measures, there was a dose-response with the effect being more pronounced in the patients who received the TID dosing regimen (TID versus placebo, p=0.02 for Detection and p=0.01 for LFT).

A positive cognitive effect of neflamapimod in the TID dosing regimen was also seen for the Mini-Mental Status Examination (MMSE) at week 8, which is the first on-treatment timepoint the MMSE was administered. The mean change from baseline to week 8 in MMSE, where an increase represents improvement, was -0.43 in the placebo group and +1.07 in neflamapimod TID patients (p=0.033 for difference) (Table 2). There was also a dose-response for MMSE at week 8, with a dose-trend analysis revealing a significant dosing regimen dependent treatment effect (p=0.041 by Jonckheere-Terpstra statistical test).

**Table 1. Mean change (SD) from baseline to Week 4 in cognitive measures by treatment group.**

| Test | Placebo | NFNM |
|---|---|---|
| Detection | +0.024 | -0.024 |
| Letter Fluency Test | -1.7 | +3.0 |

**Table 2. Mean change (SD) from baseline to Week 8 in cognitive measures by treatment group.**

| Test | Placebo | NFNM |
|---|---|---|
| MMSE | -0.43 | +1.07 |

The combined results, particularly given the dose-response, indicate that neflamapimod has a significant beneficial effect on cognition in patients with dementia with Lewy bodies.

## Claims

1. A selective p38α mitogen activated protein kinase (MAPK) inhibitor for use in the treatment of Dementia with Lewy Bodies (DLB), the use comprising administering to a subject having DLB said selective p38α mitogen activated protein kinase (MAPK) inhibitor, wherein said use reverses and/or slows the progression of DLB.

2. The selective p38α mitogen activated protein kinase (MAPK) inhibitor for use according to claim 1, wherein said reversal and/or slowing of the progression of DLB is **characterized by** the inhibition of the loss of cholinergic neurons in the medial septum of the subject.

3. The selective p38α mitogen activated protein kinase (MAPK) inhibitor for use according to any one of claims 1-2, wherein the selective p38α mitogen activated protein kinase (MAPK) inhibitor is neflamapimod.

4. The selective p38α mitogen activated protein kinase (MAPK) inhibitor for use according to any of the preceding claims, wherein the subject has alpha synuclein deposits in the hippocampus.

## Patentansprüche

1. Selektiver p38α-Mitogen-aktivierter Proteinkinase (MAPK)-Inhibitor zur Verwendung in der Behandlung von Lewy-Körper-Demenz (DLB), wobei die Verwendung die Verabreichung des selektiven p38α-Mitogen-aktivierten Proteinkinase (MAPK)-Inhibitors an einen Patienten mit DLB umfasst, wobei die Verwendung das Fortschreiten von DLB umkehrt und/oder verlangsamt.

2. Selektiver p38α-Mitogen-aktivierter Proteinkinase (MAPK)-Inhibitor zur Verwendung gemäß Anspruch 1, wobei die Umkehrung und/oder Verlangsamung des Fortschreitens von DLB durch die Hemmung des Verlusts cholinerger Neuronen im medialen Septum des Patienten gekennzeichnet ist.

3. Selektiver p38α-Mitogen-aktivierter Proteinkinase (MAPK)-Inhibitor zur Verwendung gemäß einem der Ansprüche 1-2, wobei der selektive p38α-Mitogen-aktivierte Proteinkinase (MAPK)-Inhibitor Neflamapimod ist.

4. Selektiver p38α-Mitogen-aktivierter Proteinkinase (MAPK)-Inhibitor zur Verwendung gemäß einem der vorstehenden Ansprüche, wobei der Patient Alpha-Synuclein-Ablagerungen im Hippocampus aufweist.

## Revendications

1. Un inhibiteur sélectif de la protéine kinase activée par le mitogène p38α (MAPK) destiné à être utilisé dans le traitement de la démence à corps de Lewy (DLB), l'utilisation comprenant l'administration à un sujet atteint de DLB dudit inhibiteur sélectif de la protéine kinase activée par le mitogène p38α (MAPK), dans lequel ladite utilisation inverse et/ou ralentit la progression de la DLB.

2. L'ínhibiteur sélectif de la protéine kinase activée par le mitogène p38α (MAPK) destiné à être utilisé selon la revendication 1, dans lequel ladite inversion et/ou ralentissement de la progression de la DLB est **caractérisé par** l'inhibition de la perte de neurones cholinergiques dans le septum médian du sujet.

3. L'inhibiteur sélectif de la protéine kinase activée par le mitogène p38α (MAPK) destiné à être utilisé conformément à l'une quelconque des revendications 1 à 2, dans lequel l'inhibiteur sélectif de la protéine kinase activée par le mitogène p38α (MAPK) est le néflamapimod.

4. L'inhibiteur sélectif de la protéine kinase activée par le mitogène p38α (MAPK) destiné à être utilisé selon l'une quelconque des revendications précédentes, dans lequel le sujet présente des dépôts d'alpha-synucléine dans l'hippocampe.
